Europäisches Patentamt

European Patent Office

Office européen des brevets

(11) Publication number: **0 175 274**
**B1**

(12) **EUROPEAN PATENT SPECIFICATION**

(45) Date of publication of patent specification: **22.11.90**

(21) Application number: **85111490.0**

(22) Date of filing: **11.09.85**

(51) Int. Cl.⁵: **A 61 J 1/00**

(54) **Method of separation of blood components.**

(30) Priority: **13.09.84 JP 190476/84**

(43) Date of publication of application:
**26.03.86 Bulletin 86/13**

(45) Publication of the grant of the patent:
**22.11.90 Bulletin 90/47**

(84) Designated Contracting States:
**BE CH DE FR GB IT LI NL SE**

(56) References cited:
**EP-A-0 073 847**
**WO-A-84/00492**
**FR-A-2 283 700**
**US-A-3 870 042**

(73) Proprietor: **TERUMO KABUSHIKI KAISHA
trading as TERUMO CORPORATION
44-1, 2-chome, Hatagaya Shibuya-Ku
Tokyo 151 (JP)**
(73) Proprietor: **Japanese Red Cross Society
1-3, 1-chome Shibadaimon
Minato-ku Tokyo (JP)**

(72) Inventor: **Tomono, Tsugikazu
379-30, Toyoshiki
Kashiwa-shi Chiba-ken (JP)**
Inventor: **Igarashi, Shigeru
22-10, 5-chome Yahiro
Sumida-ku Tokyo (JP)**
Inventor: **Sawada, Eiko
9-5, 4-chome Ebisu
Shibuya-ku Tokyo (JP)**
Inventor: **Tokunaga, Eiichi
1-6, 1-chome Saginomiya
Nakano-ku Tokyo (JP)**
Inventor: **Minagawa, Yoshinori
1897-95, Aoki
Fujinomiya-shi Shizuoka-ken (JP)**
Inventor: **Kawaoka, Tatsuhiko
237-97, Hoshiyama
Fujinomiya-shi Shizuoka-ken (JP)**

**EP 0 175 274 B1**

(74) Representative: **Henkel, Feiler, Hänzel und Partner**
**Möhlstrasse 37**
**D-8000 München 80 (DE)**

## Description

This invention relates to a method of separation of blood components. More particularly, this invention relates to a method of efficient separation of blood components such as blood coagulating factor VIII from blood plasma.

Hemophilia is an inherited disorder of the blood marked by a permanent tendency to hemorrhages due to the lack of a blood coagulating activity of factor VIII or factor IX. The hemophilia due to deficiency of factor VIII is called hemophilia A (factor VIII deficiency, classic hemophilia) and the hemophilia due to deficiency of factor IX is called hemophilia B (factor IX deficiency, Christmas disease). Unfortunately, no radical therapy has yet been established for hemophilia. The conventional therapy inevitably resorted to even today consists in supplementing factor VIII to combat hemophilia A or factor IX, hemophilia B, as occasion demands. When a patient of hemophilia has a hemorrhage, the supplementation of a deficient coagulation factor is to be started with minimum loss of time. When the patient has to undergo a surgical operation or a dental treatment, this supplementation is to be made in advance of the operation or treatment. The factor VIII is instable and loses in activity during storage. Thus, the supplementation of the factor VIII necessitates use of fresh blood. The factor IX is to be stable that the supplementation of this factor may be effected by use of preserved blood. When the whole blood or the blood plasma is used as it is, ample supply of a coagulation factor is obtained with great difficulty. The intravenous injection of a concentrated preparation such as a cryoprecipitate, Cohn fraction I, or a concentrated factor VIII for the factor VIII or that of a refined prothrombin for the factor IX has found popular acceptance.

In the preparation of factor VIII, the concentrated preparation of factor VIII is obtained by highly concentrating the factor VIII. The recovery of this concentrate is only about 20% because the coagulating activity of the factor VIII is degraded during the course of the concentration. Further since the blood plasma used as the raw material for this preparation is collected from many donors, the possibility of viruses such as those responsible for hepatitis mingling into the preparation is high. In contrast, the cryoprecipitate is prepared by a simple and convenient procedure from the blood plasma collected from a small number of donors and, therefore, the possibility of viruses mingling into the product is low. Moreover, the recovery of the coagulating activity of the factor VIII is high. Since this preparation is obtained by concentration of a limited degree, however, it has a large liquid content and contains fibrinogen and other extraneous proteins in a large proportion. When it is transfused in a large amount, therefore, it has the possibility of increasing the amount of blood plasma for circulation, elevating the concentration of fibrinogen, and inducing secondary reactions such as hemolysis.

For elimination of the disadvantages suffered by the conventional preparation, efficient removal of fibrinogen from cryoprecipitate without inactivation of the factor VIII is important. No satisfactory means of materializing this efficient removal of extraneous proteins has been proposed to date.

Prior art document US—A—3870042 discloses a method for separating and injecting a blood component similar to the method described in the first part of claim 1. This method uses a system of blood-taking and decanting pockets interconnected by tubes, a storage pocket, the dimensions of which correspond to the amount of component to be injected, a filter and injection means in this storage pocket. The method is useful for separating by cryo-precipitation and for injecting into a patient the factor VIII used against hemophilia.

An object of this invention, therefore, is to provide a novel method for the separation of blood components.

Another object of this invention is to provide a novel method for the separation of the factor VIII, which combines separation of cryoprecipitate from blood plasma and removal of fibrinogen from the cryoprecipitate.

A further object of this invention is to provide a method for obtaining a preparation of factor VIII of low extraneous protein content by effecting efficient separation of the factor VIII from the blood plasma originating in a small number of donors by a simple and convenient procedure.

This object is solved by a method as defined in claim 1.

An apparatus for the separation of blood components comprises a blood plasma container connected to one end of a transfer tube for blood plasma, a cryoprecipitate-deficient blood plasma container connected in a state capable of communication to the blood plasma container through a tube for transferring a cryoprecipitate-deficient blood plasma separated in the blood plasma container, and a container having connecting means and holding therein a cryoprecipititate dissolving agent and connected in a state capable of communication to the blood plasma container by the connecting means.

Another apparatus for the separation of blood components uses as the connecting means a tube having a needle at one end thereof and connected at the other end thereof to the container for holding the cryoprecipitate dissolving agent. A further apparatus for the separation of blood components has the connecting means provided with flow path closing means capable of establishing communication. Another apparatus for the separation of blood components has the flow path closing means provided with a breakable part adapted to be broken for the purpose of opening a flow path therethrough. A further apparatus for the separation of blood components has the blood plasma container and the cryoprecipitate-deficient blood plasma container interconnected to each other through the medium of the tube for the transfer of cryoprecipitate-deficient blood plasma. Another apparatus for the separation of blood components has the blood plasma container and the cryoprecipitate-deficient blood plasma container adapted so as to be interconnected to each other through the medium of the tube for the transfer of cryoprecipitate-deficient blood plasma. A further apparatus for the separation of blood components uses as the cryoprecipitate dissolving agent a neutral aqueous solution at least one substance selected from the group consisting of water-soluble amino acids such as glycine, lysine, alanine, and glutamine, saccharides such as sucrose, glucose, and mannitol, and neutral salts such as sodium chloride and potassium chloride.

A container includes connecting means for connecting to other containers in a state capable of communication to said other containers, and holding therein a cryoprecipitate dissolving agent comprising a neutral aqueous solution containing at least one substance selected from the group consisting of water-soluble amino acids, saccharides, and neutral salts, as a stabilizer for the blood coagulating factor VIII.

The expression "connected in a state capable of communication" as used in the present specification means the manner in which two containers are interconnected to each other with a tube incorporating in flow path thereof such flow path closing means as is provided with a breakable part adapted to be broken for the purpose of opening flow path therethrough and the manner in which the two containers are interconnected to each other through the medium of a tube provided at one end thereof with a pin or needle.

The invention will now be described in greater detail in conjunction with the attached drawings, in which:

Fig. 1 through Fig. 7 are front views illustrating typical apparatuses for the separation of blood components.

Figs. 8 and 9 are sectional views illuatrating typical flow path closing means capable of establishing communication, and

Fig. 10 is a sectional view illustrating a typical mixing part.

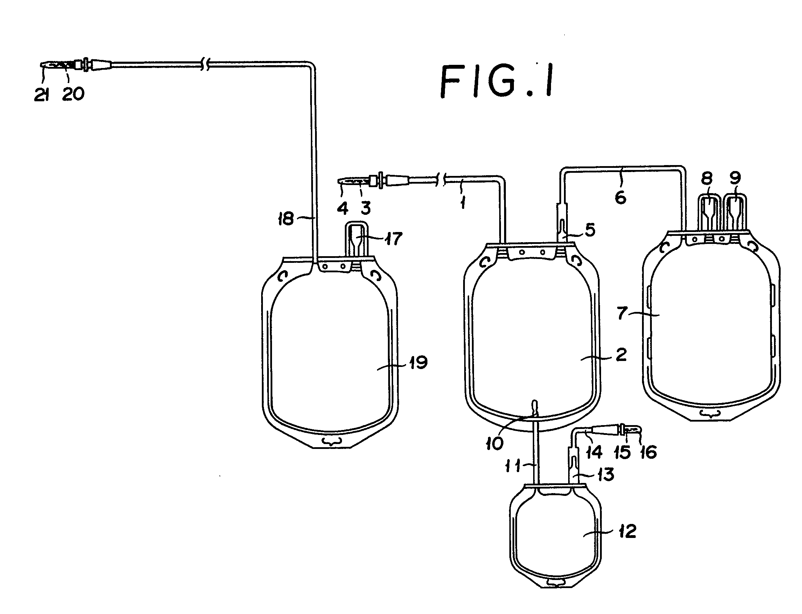

Fig. 1 depicts a typical apparatus for the separation of blood components. To one end of a blood plasma inlet tube 1 is connected a blood plasma container 2. A vial needle 3 is connected when necessary to the other end of the inlet tube 1. The vial needle 3 is covered with a cap 4. This blood plasma container 2 is connected to a cryoprecipitate-deficient blood plasma container 7 through the medium of a cryoprecipitate-deficient blood plasma transfer tube 6 provided with flow path closing means 5 capable of establishing communication. This cryoprecipitate-deficient blood plasma container 7 is optionally provided with mixing ports adapted to be pierced with vial needles for the purpose of establishing communication with the interior of the container 7. These mixing ports are covered with fracturable tightly sealing members 8 and 9. The blood plasma container 2 is connected to a cryoprecipitate dissolving agent container 12 through the medium of a cryoprecipitate dissolving agent feed tube 11 which is means of communication provided with flow path closing means 10 capable of communication. The container 12 holds therein a cryoprecipitate dissolving agent. To this container 12 is connected a discharge tube 14 provided with a flow path closing means 13 capable of communication. The discharge tube 14 has a male tapered connector 15 attached to the leading end thereof. This container 15 is covered with a cap 16.

Preparatorily to use, the blood plasma container 2 is connected when necessary through the medium of the blood plasma inlet tube 1 to a blood container 19 which is provided with a mixing port covered with a fracturable tightly sealing member 17 and a blood inlet tube 18. The blood inlet tube 18 has a vial needle 20 connected to the leading end thereof. This vial needle 20 is covered with a cap 21.

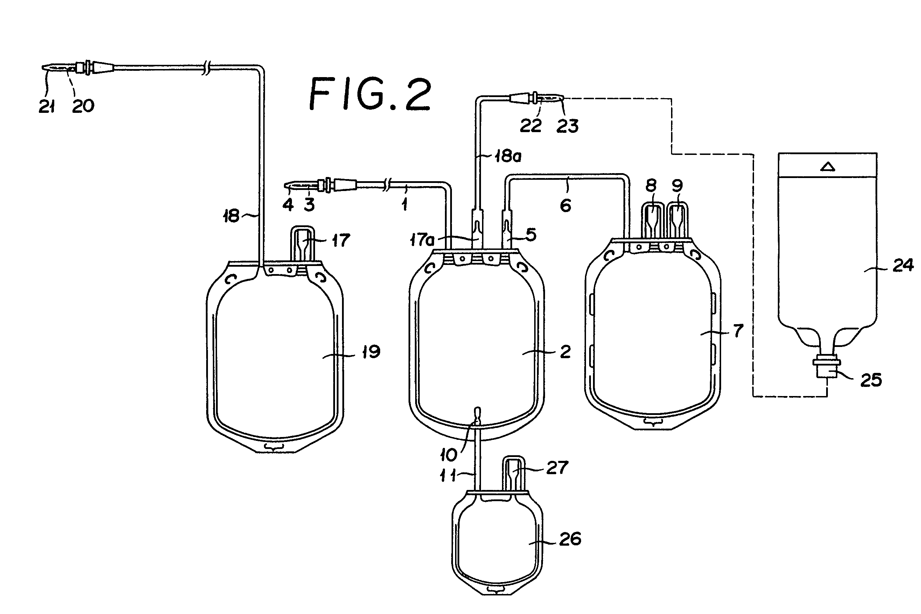

In the apparatus for the separation of blood components shown in Fig. 2 and constructed as illustrated in Fig. 1, a cryoprecipitate dissolving agent feed tube 18a provided with flow path closing means 17a capable of communication is further connected to the blood plasma container 2. Connected to the leading end of the feed tube 18a is a vial needle 22, which is covered with a cap 23. The container 26 is normally kept empty. In Fig. 2, the numeral symbols which have equivalents in Fig. 1 denote identical parts. When the dissolving agent container is not intended to hold the cryoprecipitate solution repeatedly as in the apparatus of Fig, it is not specifically limited in form. It may be in the form of a syringe or a filled vial, for example.

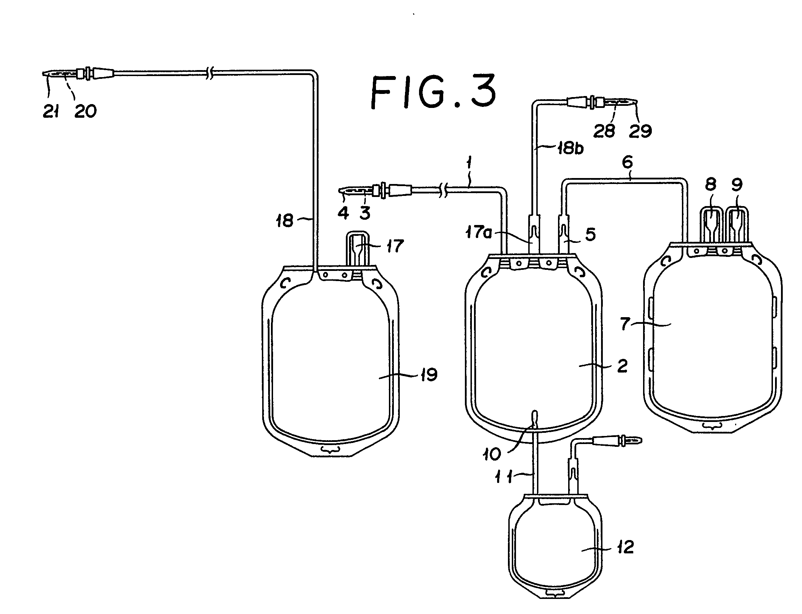

In the apparatus of Fig 3, the cryoprecipitate dissolving agent container 12 is not provided with any mixing port and is filled with a cryoprecipitate dissolving agent from the beginning. In the diagram, 18b denotes a blood coagulating factor VIII discharge tube. To the leading end of this tube 18b is connected a male connector 28, which is covered with a cap 29. The numerical symbols of Fig. 3 which have equivalents in Fig. 2 denote identical parts.

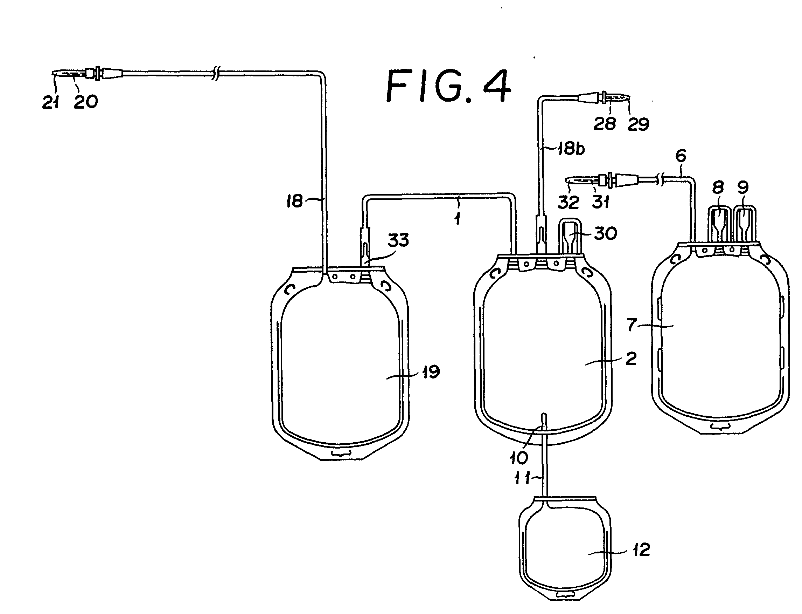

In the apparatus for the separation of blood components shown in Fig. 4 and constructed as illustrated in Fig. 3, the cryoprecipitate-deficient blood plasma transfer tube 6 is not connected in advance to the blood plasma container. To the leading end of the transfer tube 6 is attached a vial needle 31, which is covered with a cap 32. The blood plasma container 2 is provided with a mixing port, which is covered with a fracturable tight sealing part 30. In the apparatus of Fig. 4, the blood plasma container 2 and the blood container 19 are interrconnected to each other through the medium of flow path closing means 33 capable of communication. The numerical symbols of Fig. 4 which have equivalents in Fig. 1 denote identical parts.

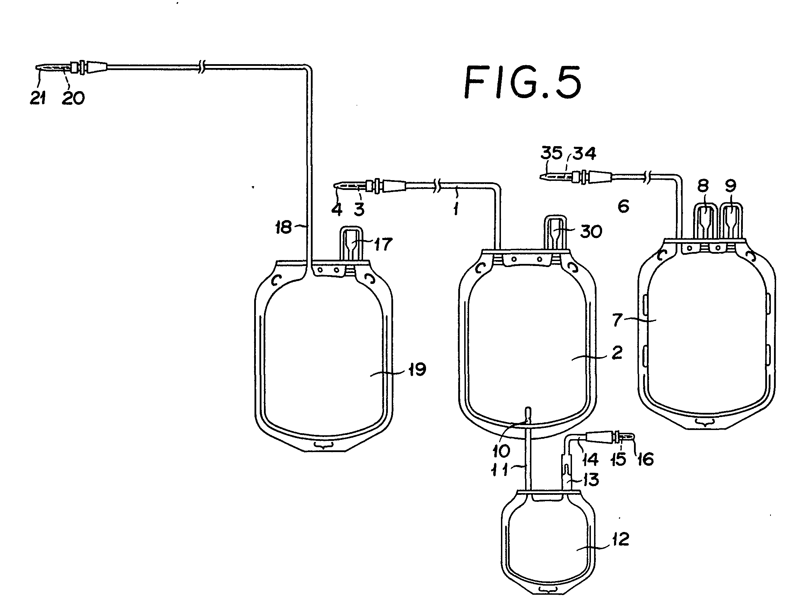

In the apparatus for the separation of blood components shown in Fig. 5 and constructed as illustrated in Fig. 1, the cryoprecipitate-deficient blood plasma trasfer tube 6 is not connected in advance to the blood plasma container 2. To the leading end of this transfer tube 6 is attached a vial needle 34, which is covered with a cap 35. The numerical symbols of Fig. 5 which have equivalents in Figs. 1 and 4 denote identical parts.

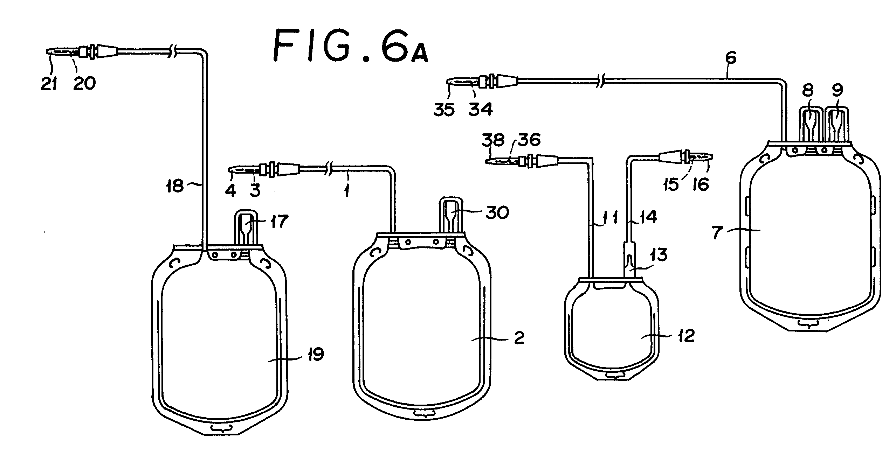

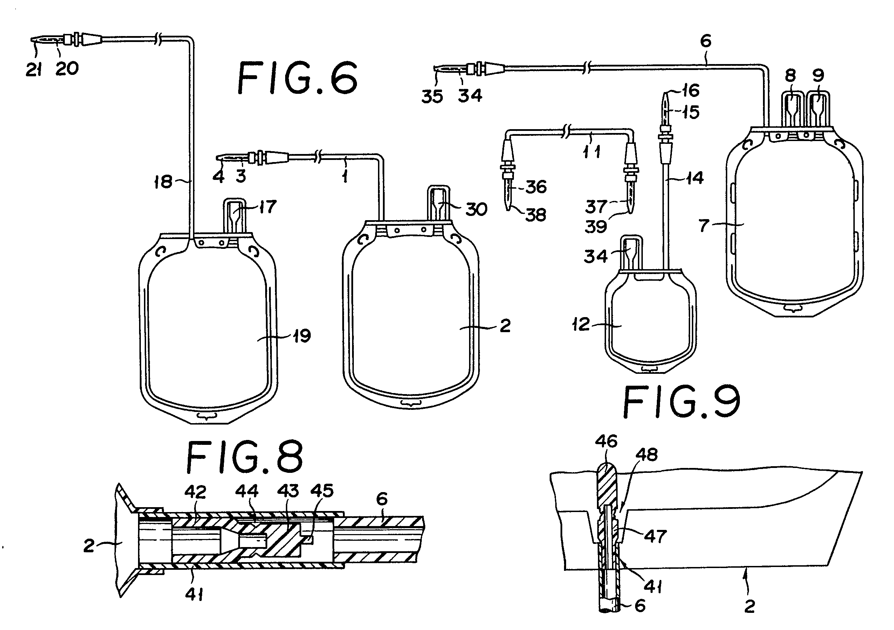

In the apparatus for the separation of blood components shown in Fig. 6 and constructed as illustrated in Fig. 1, the blood plasma container 2 and the cryoprecipitate dissolving agent container 12 are not interconnected in advance to each other. The cryoprecipitate dissolving agent container 12 is provided with a mixing port, which is covered with a fracturable tightly sealing part 34. The cryoprecipitate dissolving agent transfer tube 11 is independently formed and has severally attached to the opposite ends thereof vial needles 36 and 37, which are covered respectively with caps 38, 39. The numerical symbols of Fig. 6 which have equivalents in Fig. 1, 4 and 5 denote identical parts.

In the apparaatus for the separation of blood components shown in Fig. 6A and constructed as illustrated in Fig. 1, the blood plasma container 2 and the cryoprecipitate dissolving agent container 12 are not interconnected in advance to each other. The cryoprecipitate dissolving agent container 12 is connected to a discharge 14 provided with a flow path closing means 13 capable of communication, and the discharge tube 14 has a male tapered connector 15 attached to the leading end thereof. The cryoprecipitate dissolving agent transfer tube 11 is attached to the end of vial needles 36, which are covered with cap 38. The numerical symbols of Fig. 6 which have equivalents in Fig. 1, 4 and 5 denote identical parts.

In the apparatus for the separation of blood components shown in Fig. 7 and constructed as illustrated in Fig. 1, the blood plasma container 2 is connected to one end of the blood plasma inlet tube 1 and the vial needle 3 is connected when necessary to the other end. This vial needle 3 is covered with the cap 4. The blood plasma container 2 is connected to the cryoprecipitate-deficient blood plasma container 7 through the medium of the cryoprecipitate-deficient blood plasma transfer tube 6 which is provided with the flow path closing means 5 capable of communication. This cryoprecipitate-deficient blood plasma container 7 is optionally provided with mixing ports, which are covered with fracturable tight sealing parts 8 and 9. The blood plasma container 2 is connected to the cryoprecipitate dissolving agent container 12 through the medium of the cryoprecipitate dissolving agent feed tube 11 which is a connecting means provided with flow path closing means 10a capable of communication. The container 12 holds therein a cryoprecipitate dissolving agent. Further to this container is connected the discharge tube 14 provided with the flow path closing means 13 capable of communication. To the leading end of the discharge tube 14 is attached the male tapered connector 15, which is covered with the cap 16. The numerical symbols of Fig. 7 which have equivalents in Fig. 1 denote identical parts.

In the above apparatuses, a plurality of tubes are directly extended from one container. This arrangement does not constitute any essential requirement. Optionally, the plurality of tubes may be ramified through a branched tube from one tube.

In the apparatuses described above, the containers and the tubes are usually made of flexible synthetic resin, preferably flexible vinyl chloride resin. The length of the connecting means (tube) for transfer of the dissolving agent is desirably not more than 50 cm, preferably not more than 25 cm. The inside diameter of the connection means is desired to fall in the range of 1.0 to 3.0 mm. By selecting the dimensions of the connection means as described above, the portion of the dissolving agent or the cryoprecpitate solution which, while in transit, is retained inside the tube and consequently kept from effectively filling its part can be decreased and the transfer itself can be smoothened.

As flow path closing means capable of communication, there can be used any means which normally keeps the flow path closed and, when the flow path is needed, opens the closing member and forms a path for liquid. Preferably this means is provided with a breaking part which is adapted to be broken for the purpose of opening a flow path. For example in Fig. 8, it comprises a hollow tube 42 having an outside diameter substantially equal to the inside diameter of a liquid port and having gradually convering outer end and a solid cylinder 43 formed integrally at the leading end of the hollow tube 42 and having an outside diameter smaller than the inside diameter of the liquid port and larger than the inside diameter of the tube (tube 6, for example), as illustrated in Fig. 8, both made of rigid synthetic resin such as, for example rigid vinyl chloride resin. It is fastened to the inner wall of the liquid part 41. This flow path closing means is provided with a notch 44 near the boundary between the hollow tube 42 and the cylinder 43 on the hollow tube 42 side. Before use, the solid cylinder 43 obstructs communication between the container (container 2, for example) and the tube (tube 6, for example). Preparatory to use, the solid cylinder 43 is cut at the notch 44 with the force exerted externally as with finger tips to establish communication through the interior of the hollow tube 42 between the container and the tube. The solid cylinder 43 is provided when necessary on the end face thereof with a protrusion 45 of the shape of a flat plate having a width equal to the outside diameter of the cylinder, so that the solid cylinder 43 after separation from the hollow tube 42 may be prevented from closing the communicating tube.

Fig. 9 depicts another typical flow path closing means. In this case, the flow path closing means is provided in the liquid port 41 formed in the container (container 2, for example), specifically on the container's interior side. It comprises a tube 47 having an outside diameter substantially equal to the inside diameter of the liquid port 41 and a cylinder 46 formed integrally at the leading end of the tube 47, both made of rigid synthetic resin such as, for example, rigid vinyl chloride resin. A notch 48 is formed between the cylinder 46 and the tube 47. Before use, the cylinder obstructs flow of liquid. Preparatory to use, the cylinder 46 is bent and cut off at the notch 48 by the force exerted externally as with finger tips so as to establish communication between the container (container 2 for example) and the tube (tube 6, for example).

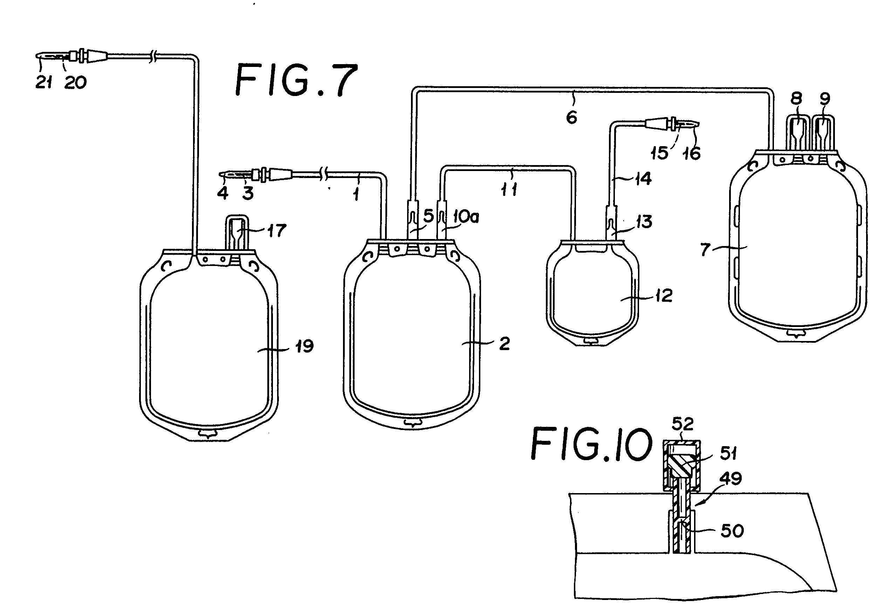

In Fig. 10, the liquid port 49 is provided halfway along the length of its interior with a partition wall 50 adapted to block the path. Necessary flow of liquid is obtained by stripping a rubber member 51 of a cover 52, piercing the rubber member 51 with a vial needle, and forcing the vial needle through the partition wall 50. When the vial needle is extracted from the partition wall 51, the rubber member 51 obstructs otherwise inevitable backflow of the liquid. Optionally, the liquid port 49 may be closed with a fracturable tight sealing member instead of incorporating the rubber member 51 and wrapping the rubber member with the cover 52.

In the case of the apparatus for the separation of blood components illustrated in Fig. 1, the blood collected from donors is introduced through a collection needle 21 and a tube 18 into the blood container 19 and the blood in the container 19 is centrifuged to be separated into blood cell portion and blood plasma portion. Then the blood plasma portion is introduced via the blood plasma inlet tube 1 into the blood plasma container 2, frozen, then thawed, and centrifuged to be separated into a cryoprecipitate-deficient blood plasma layer and a cryoprecipitate layer. The cryoprecipitate-deficient blood plasma which forms the supernatant is then transferred via the cryoprecipitate-deficient blood plasma transfer tube 6 into the cryoprecipitate-deficient blood plasma container 7. The container 7 now holding the blood plasma therein is separated from the apparatus and put to use for the production of some other blood plasma preparation. Then, the transfer tube 6 is sealed and cut at a proper portion thereof and the flow path closing means 10 is manipulated to establish a flow path. From the cryopreciptate dissolving agent container 12, a cryoprecipitate dissolving agent which is formed of one member or a mixture of two or more member selected from the group consisting of water-soluble amino acids such as glycin, lysine, alanine, and glutamine, saccharides such as sucrose, glucose, and mannitol, and neutral salts such as sodium chloride and potassium chloride is fed via the cryoprecipitate dissolving agent feed tube 11 to the blood plasma container 2. The solution of cryoprecipitate which is obtained inside the container 2 is returned to the dissolving agent container 12. The tube 11 is sealed at a proper portion thereof as with a pinch and cut there. The container 12 now separated from the apparatus is heated to a prescribed temperature (falling in the range of 40° to 70°C, for example) and centrifuged. A defibrinated cryoprecipitate (blood coagulating factor VIII) which is consequently formed as the upper layer of modified protein fraction is separated and discharged through the discharge tube 14.

The discharged factor VIII is filtered such as through a filter, for example, to be freed from impurities and then transferred into a vial to be freeze dried and put to use in the production of some preparation. Further, in the above mentioned operation, cryoprecipitate in the blood plasma container 2 may be charged directly to the cryoprecipitate dissolving agent container 12 through the tube 11 and mixed with the dissolving agent to dissolve the cryoprecipitate into the dissolving agent.

In the case of the apparatus for the separation of blood components illustrated in Fig. 2, after the cryoprecipitate-deficient blood plasma has been removed similarly to the apparatus of Fig. 1, the cryoprecipitate dissolving agent held in advance in the cryoprecipitate container 24 is fed via the feed tube 18a into the blood plasma container 2. The solution which is consequently obtained in the container 2 is transferred into the container 26 which has been empty so far, there to be heated and subsequently centrifuged. The defibrinated cryoprecipitate consequently obtained is discharged for separation through the mixing port 27 after the mixing port has been exposed by breakage of the fructurable tight sealing member 27.

In the case of the apparatus for the separation of blood components illustrated in Fig. 3, the dissolving agent from the dissolving agent container 12 is fed to the blood plasma container 2. This container 2 with its content is subjected to centrifugation. Then, the defibrinated cryoprecipitate is discharged for separation through the discharge tube 18b.

In the case of the apparatus for the separation of blood components illustrated in Fig. 4, after the blood plasma has been centrifuged similarly to the apparatus of Fig. 3, the vial needle 31 at the leading end of the transfer tube 6 is thrust through the mixing port inside the fracturable tight sealing member 30. From this point onward, the procedure of the apparatus of Fig. 3 is followed to discharge the defibrinated cryoprecipitate through the discharge tube 18b.

In the case of the apparatus for the separation of blood components illustrated in Fig. 5, after the blood plasma has been centrifuged similarly to the apparatus of Fig. 1, the vial needle 34 at the leading end of the transfer tube 6 is thrust through the mixing port within the fracturable tight sealing member 30. From this point onward, the procedure of the apparatus of Fig. 3 is followed to discharge the defibrinated cryoprecipitate through the discharge tube 14.

In the case of the apparatus for the separation of blood components illustrated in Fig. 6, the cryoprecipitate-deficient blood plasma container 7 is separated from the blood plasma container 2 from the beginning. These containers are interlocked to each other only when the cryoprecipitate-deficient blood plasma is separated and removed. The dissolving agent tank 12 is separated from the blood plasma container 2 from the beginning. They are connected to each other via the transfer tube 11 when the cryoprecipitate dissolving agent is supplied. In all the other respects, the operation of the apparatus is similar to that of the apparatus of Fig. 1.

In the case of the apparatus for the separation of blood components illustrated in Fig. 7, the blood plasma is introduced through the blood plasma inlet tube 1 into the blood plasma container 2, frozen, then thawed, and centrifuged to be separated into the cryoprecipitate-deficient blood plasma layer and the cryoprecipitate layer. The cryoprecipitate-deficient blood plasma forming the supernatant is transferred via the cryoprecipitate-deficient blood plasma transfer tube 6 into the cryoprecipitate-deficient blood plasma container 7. Then, the transfer tube 6 is cut. From the cryoprecipitate dissolving agent container 12, the cryoprecipitate dissolving agent is fed through the cryoprecipitate dissolving agent feed tube 11 into the blood plasma container 2. The solution consequently obtained in the container 2 is returned to the dissolving agent container 12, heated to a prescribed temperature, and then centrifuged. The defibrinated cryoprecipitate (factor VIII) formed as the upper layer of degenerated protein fraction is discharged out of the discharge tube 14.

Now, the present invention will be described more specifically below with reference to a working example.

Example:
Fresh blood, 200 or 400 ml in volume, was collected through a collection needle 21 and a tube 18 into a blood container 19 and then centrifuged to be separated into a blood plasma component and a blood cell component. The blood plasma in the supernatent was transferred into other separate bag (not shown). Then, in an apparatus for the separation of blood components illustrated in Fig. 1, the blood plasma 400 ml in volume was introduced from the separate bag into a blood plasma container 2 through a blood plasma inlet tube 1, frozen at −70°C, and then suddenly thawed in a constant temperature bath at 2° to 4°C. The container 2 containing the blood plasma was set in place on a centrifugal separator and centrifuged at 3,000 G for 20 minutes. By transferring the cryoprecipitate-deficient blood plasma in the supernatent through the transfer tube 6 into a cryoprecipitate-deficient blood plasma container 7, 7 ml of cryoprecipitate was obtained in the blood plasma container 2. Subsequently, from a cryoprecipitate dissolving agent container 12, 15 ml of a dissolving agent container 13.3 mg of glycin and 4 mg of an aqueous sodium chloride solution each per ml was fed through a dissolving agent feed tube 11 into the blood plasma container 2 to effect solution of the cryoprecipitate in the sodium chloride solution of glycin. The resultant solution was returned to the dissolving agent container 12, heated at 54°C for 5 minutes, and then centrifuged at 1,800 G for 20 minutes. When the supernatant consequently formed was discharged through a discharge tube 14, 18 ml of defibrinated cryoprecipitate containing factor VIII in a concentration of 16 IU/ml was obtained in the supernatant.

An apparatus for the separation of blood components comprises a blood plasma container connected to one end of the blood plasma inlet tube, a cryoprecipitate-deficient blood plasma container connected in a state capable of communication to the blood plasma container through the medium of a tube for transferring a cryoprecipitate-deficient blood plasma separated in the blood plasma container, connecting means provided with a flow path for liquid and connected in a state capable of communication to the blood plasma container, and a container for holding therein a cryoprecipitate dissolving agent to be injected through the connecting means into the blood plasma container. The apparatus, therefore, is capable of separating from blood plasma, cryoprecipitate containing factor VIII in a very high concentration and deprived of fibrinogen, a substance contained in a large amount in cryoprecipitate and constituting a cause for secondary reactions during volume transfer of factor VIII.

When the blood plasma container, the cryoprecipitate-deficient blood plasma container, and the cryoprecipitate dissolving agent container are interconnected to one another in advance, the apparatus as a whole is enabled to form a closed system and has no possibility of causing air pollution during manipulation of tubes connecting the containers. Moreover, the apparatus is operated with great ease. When the cryoprecipitate dissolving agent container is formed independently, this container can be autoclaved for the purpose of sterilization and the other parts of the apparatus can be sterilized with gas or radiation other than by means of autoclaving. Thus the apparatus can be manufactured with ease. When the cryoprecipitate dissolving agent container is located below the blood plasma container, the cryoprecipitate dissolving agent injected from the lower container into the upper container immediately comes into contact with the cryoprecipitate and, therefore, the solution of the cryoprecipitate is effected quickly. Further, the transfer of the dissolved cryoprecipitate into the aforementioned container can be easily effected by virtue of gravitational attraction.

**Claims**

1. A method for separation of blood components comprising the steps of:
   centrifuging blood in a centrifuging container to separate a blood cell portion and a blood plasma portion,
   freezing said blood plasma portion,
   thawing said blood plasma portion,

centrifuging said blood plasma portion in a blood plasma container (2) to separate a cryoprecipitate-deficient blood plasma layer and a cryoprecipitate layer, and

adding a cryoprecipitate dissolving agent into said cryoprecipitate layer thereby forming a cryoprecipitate solution,

characterized by comprising steps of:

heating said cryoprecipitate solution, and

centrifuging said cryoprecipitate solution in a dissolving agent container (12) to separate therefrom a defibrinogen-cryoprecipitate.

2. A method according to claim 1, wherein said method includes feeding said cryoprecipitate dissolving agent from the cryoprecipitate dissolving agent container (12) to said cryoprecipitate layer and recycling said cryoprecipitate solution to said dissolving agent container (12).

3. A method according to claim 1, wherein said method includes feeding said cryoprecipitate dissolving agent from a cryoprecipitate dissolving agent container (12) to said cryoprecipitate layer and transferring said cryoprecipitate solution to another container for centrifuging.

4. A method according to claim 1, wherein said method includes feeding said cryoprecipitate dissolving agent from a cryoprecipitate dissolving agent container (12) to said cryoprecipitate layer in said blood plasma container (2) and said cryoprecipitate solution is centrifuged in said blood plasma container (2).

**Patentansprüche**

1. Verfahren zur Auftrennung von Blutkomponenten, umfassend folgende Stufen:

Zentrifugieren von Blut in einem Zentrifugenbehälter zur Trennung eines Blutkörperchenanteils und eines Blutplasmaanteils;

Gefrieren des Blutplasmaanteils;

Auftauen des Blutplasmaanteils;

Zentrifugieren des Blutplasmaanteils in einem Blutplasmabehälter (2) zur Abscheidung einer Blutplasmaschicht mit einem Mangel an Kryoausfällung und einer Kryoausfällungsschicht und

Zugeben eines die Kryoausfällung auflösenden Mittels zu der Kryoausfällungsschicht zur Bildung einer Kryoausfällungslösung,

gekennzeichnet durch folgende (zusätzliche) Stufen:

Erwärmen der Kryoausfällungslösung und

Zentrifugieren der Kryoausfällungslösung in einem Auflösungsmittelbehälter (12) zur Abtrennung einer Defibrinogen-Kryoausfällung von der Lösung.

2. Verfahren nach Anspruch 1, dadurch gekennzeichnet, daß zusätzlich der Kryoausfällungsschicht das die Kryoausfällung auflösende Mittel aus dem (Kryoausfällungs-) Auflösungsmittelbehälter (12) zugeführt und die Kryoausfällungslösung in den Auflösungsmittelbehälter (12) rückgeführt wird.

3. Verfahren nach Anspruch 1, dadurch gekennzeichnet, daß zusätzlich der Kryoausfällungsschicht das die Kryoausfällung auflösende Mittel aus einem (Kryoausfällungs-) Auflösungsmittelbehälter (12) zugeführt und die Kryoausfällungslösung in einen weiteren Behälter zum Zentrifugieren überführt wird.

4. Verfahren nach Anspruch 1, dadurch gekennzeichnet, daß zusätzlich der Kryoausfällungsschicht in dem Blutplasmabehälter (2) das die Kryoausfällung auflösende Mittel aus einem (Kryoausfällungs-) Auflösungsmittelbehälter (12) zugeführt und die Kryoausfällungslösung in dem Blutplasmabehälter (2) zentrifugiert wird.

**Revendications**

1. Procédé de séparation des constituants du sang, comprenant les étapes suivantes:

la centrifugation du sang dans un récipient de centrifugation afin qu'une partie contenant les cellules et qu'une partie contenant le plasma sanguin soient séparées,

la congélation de la partie formant le plasma sanguin,

le dégel de la partie formant le plasma sanguin,

la centrifugation de la partie formant le plasma sanguin dans un récipient (2) de plasma sanguin afin qu'une couche de plasma sanguin déficient en cryoprécipité et une couche de cryoprécipité soient séparées, et

l'addition d'un agent de dissolution de cryoprécipité dans la couche de cryoprécipité avec formation d'une solution de cryoprécipité,

caractérisé en ce qu'il comprend les étapes suivantes:

le chauffage de la solution de cryoprécipité, et

la centrifugation de la solution de cryoprécipité dans un récipient (12) d'agent de dissolution afin qu'un cryoprécipité sans fibrine soit séparé.

2. Procédé selon la revendication 1, qui comprend l'introduction de l'agent de dissolution du cryoprécipité provenant du récipient (12) d'agent de distribution de cryoprécipité dans la couche de cryoprécipité et le recyclage de la solution de cryoprécipité dans le récipient d'agent de dissolution (12).

3. Procédé selon la revendication 1, qui comprend la transmission de l'agent de dissolution du cryoprécipité d'un récipient (12) d'agent de dissolution de cryoprécipité à la couche de cryoprécipité, et le transfert de la solution de cryoprécipité à un autre récipient pour la centrifugation.

4. Procédé selon la revenidcation 1, comprenant la transmission de l'agent de dissolution de cryoprécipité d'un récipient (12) d'agent de dissolution de cryoprécipité à la couche de cryoprécipité dans le récipient (2) de plasma sanguin, et la solution de cryoprécipité est soumise à une centrifugation dans le récipient (2) de plasma sanguin.

FIG.1

# FIG.2

FIG.3

FIG.4

FIG.5

5

FIG.6A

FIG.6

FIG.8

FIG.9

EP 0 175 274 B1

FIG.7

FIG.10

EP 0 175 274 B1